# EUROPÄISCHE PATENTSCHRIFT

(11) **EP 2 024 072 B1**
(45) Veröffentlichungstag und Bekanntmachung des Hinweises auf die Patenterteilung: **21.10.2009**
(21) Anmeldenummer: 07724494.5
(22) Anmeldetag: 24.04.2007
(51) Int. Cl.: B01F 11/00, B01F 3/04, C12M 1/02

(54) **VIBRATIONSMISCHER**
VIBRATIONAL MIXER
MÉLANGEUR VIBRATOIRE

(30) Priorität: 11.05.2006 DE 102006022306
(43) Veröffentlichungstag der Anmeldung: 18.02.2009
(73) Patentinhaber: Sartorius Stedim Biotech GmbH, 37079 Göttingen (DE)
(72) Erfinder: GRELLER, Gerhard, 37085 Göttingen (DE); REIF, Oscar-Werner, 30173 Hannover (DE)
(86) Internationale Anmeldenummer: PCT/EP2007/003564
(87) Internationale Veröffentlichungsnummer: WO 2007/131594

(56) Entgegenhaltungen:
- CH-A- 283 308
- CH-A5- 560 066
- US-A- 2 681 798
- US-A1- 2005 249 033

## Beschreibung

Die Erfindung betrifft einen Vibrationsmischer, insbesondere Einweg-Mischer zur Verwendung in Einweg-Bioreaktoren mit flexibler Wandung, bestehend aus einem von einem Antrieb in longitudinale Bewegungen versetzbaren Mischerschaft mit mindestens einem Mischelement.

Sowohl für wieder verwendbare Bioreaktoren, meist starre Behälter, als auch für Einmal-Bioreaktoren, meist flexible Beutel bzw. Behälter mit flexiblen Wänden, werden zum Mischen als auch zum Kultivieren von Organismen Vibrationsmischer verwendet. Vibrationsmischer weisen ein plattenförmiges Mischelement mit konischen Löchern (Venturi-Düsen) auf. Das Mischelement ist dabei an einem Mischerschaft angeordnet, der von einem Antrieb in longitudinale Schwingungen versetzt wird.

Ein Vibrationsmischer ist beispielsweise aus der US 2005/0249033 A1 bekannt. Dieser Einwegmischer ist in einem Einweg-Bioreaktor, einem Beutel, angeordnet.

Die CH 560 066 A5 beschreibt eine Vorrichtung zum Mischen von Flüssigkeiten, die aus einem in Vibrationsbewegungen in vertikaler Richtung versetzbaren Rührorgan besteht, welches als Mischelemet eine ebene Platte mit Durchlassöffnungen besitzt. Dabei ist der äußere Rand der Platte mit einem nach unten verlaufenden verlängerten Rand versehen, wodurch eine wesentliche Rührleistungssteigerung eintreten soll.

In der US-A 2 681 798 wird ein Vibrationsmischer beschrieben, dessen Mischelement mit konischen Durchbrechungen versehen ist. Benachbart zu dem Mischelement ist der Ausgang einer Flüssigkeits- oder Gasleitung angeordnet

Nachteilig bei dem bekannten Mischer ist, dass weitere Zugänge zu dem Reaktorinnenraum zur Zuführung von gasförmigen Medien und von flüssigen Medien notwendig sind. Insbesondere sind bei solchen Bioreaktoren die Zugänge durch die Behälterwandung problematisch.

Auch aus der DE 10 2004 013 078 A1 ist ein flexibler Einweg-Bioreaktor bekannt, der einen Vibrationsmischer aufweist. Auch hier ist ein als Mischplatte ausgebildetes Mischelement in dem Reaktorinnenraum an einem Ende eines Mischerschaftes angeordnet, der durch die Behälterwandung hindurchgeführt und außerhalb des Behälterinnenraumes von einem Antrieb eines Vibrationsmischers in longitudinale Bewegungen versetzt wird.

Die US 2005/249033 A1 beschreibt einen Vibrationsmischer in einem Beutel zum Einmalgebrauch, bei dem der Mischerschaft mit dem Beutel verbunden ist. Rotationen des Mischerschafts sind nicht erforderlich

Auch diese Mischer weisen die o.g. Nachteile auf.

In CH 283 308 A wird ein Vibrationsmischer offenbart, bestehend der aus einem von einem Antrieb in longitudinale Bewegungen versetzbaren Mischerschaft mit mindestens einem Mischelement, wobei in dem Mischerschaft ein Kanal mit einer Mündung zu einem Reaktorraum hin angeordnet ist. Nachteilig ist, dass bei Zufuhr von Gasen über den Kanal Verunreinigungen in den Reaktorraum gelangen können Aufgabe der vorliegenden Erfindung ist es daher, die Zugänge zu einem Bioreaktor zu verringern bzw. zu verbessern.

Diese Aufgabe wird in Verbindung mit dem Oberbegriff des Anspruches 1 dadurch gelöst, dass der Mündung des mindestens einen Kanals in dem Mischerschaft eine Begasungseinheit vorgelagert ist oder diese die Mündung bildet, wobei die Begasungseinheit zur Gasfilterung eine poröse Struktur aufweist.

Durch die Anordnung eines Kanals in dem Mischerschaft, der in den Reaktorinnenraum mündet, wird ein separater Zugang in den Reaktorinnenraum vermieden. Da der Schaft ohnehin durch die Wandung des Bioreaktors durchgeführt werden muss und damit gleichzeitig der in dem Schaft angeordnete Kanal, wird kein weiterer Zugang benötigt. Dadurch wird die Fertigung und die Gefahr von Undichtigkeiten der Behälterwandung erheblich reduziert.

Gemäß der Erfindung wird über den mindestens einen Kanal Gas zugeführt, wobei der Mündung des Kanals eine Begasungseinheit vorgelagert ist. Die Begasungseinheit kann aber auch die Mündung selber bilden.

Nach einer bevorzugten Ausführungsform der Erfindung ist die Mündung an dem dem Antrieb abgewandten freien Ende des Mischerschaftes angeordnet. Damit kann über das freie Ende des Mischerschaftes dem Reaktorinnenraum Gas zugeführt werden.

Nach einer weiteren bevorzugten Ausführungsform der Erfindung ist die Begasungseinheit dem Mischelement zum Antrieb hin vorgelagert. Es ist aber auch möglich, die Begasungseinheit zwischen zwei Mischelementen am Mischerschaft anzuordnen. Dabei kann über seitliche Öffnungen im Mischerschaft bzw. über die Begasungseinheit dem Reaktorinnenraum zwischen den beiden Mischelementen Gas zugeführt werden. Dies hat den Vorteil, dass das zugeführte Gas von der Strömung infolge der Mischbewegung zugeführt wird.

Gemäß einer weiteren bevorzugten Ausführungsform der Erfindung kann die Begasungseinheit als eine Membran mit mikroporöser Struktur oder auch als eine Kunststofffritte ausgebildet sein.

Gemäß einer weiteren bevorzugten Ausführungsform der Erfindung ist in dem Mischerschaft ein zweiter Kanal vorgesehen, über den Medien zu- oder abgeführt werden können.

Durch die Anordnung eines zweiten Kanals im Mischerschaft wird ein Durchgang durch die Behälterwandung, die Anordnung des Mischerschaftes sowie die Anordnung von zwei Kanälen durch die Behälterwandung hergestellt. Es wird also anstelle von drei Durchbrüchen nur ein Durchbruch in der Behälterwandung benötigt.

Nach einer weiteren bevorzugten Ausführungsform der Erfindung ist der Schaft als ein Rohr ausgebildet, dessen freies Lumen den ersten Kanal bildet. In dem freien Lumen des Schaftes kann dann ein zweites Rohr bzw. ein Schlauch angeordnet werden, der den zweiten Kanal bildet. Der Schlauch bzw. das Rohr wird dabei durch die Begasungseinheit hindurchgeführt und ist gegenüber dieser abgedichtet. Dies ermöglicht eine relativ einfache und kostengünstige Konstruktion des Mischerschaftes und seiner Kanäle.

Der Mischer kann vor bzw. nach dem Eindringen in den Bioreaktor durch Bestrahlung sterilisiert werden.

Die in den Mischerplatten vorhandenen Öffnungen (Venturi-Düsen) können nach oben bzw. nach unten verjüngt sein, wobei auch eine Kombination möglich ist.

Weitere Einzelheiten der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:
- Figur 1:: eine Seitenansicht eines in einem Bioreaktor mit flexibler Wandung angeordneten Vibrations- mischers und
- Figur 2:: eine Seitenansicht eines weiteren in einem Bioreaktor angeordneten Vibrationsmischers.

Ein Vibrationsmischer 1 besteht im Wesentlichen aus einem Mischerschaft 2 und einem Mischelement 3.

Der Vibrationsmischer 1 wird in einem Bioreaktor 4 verwendet, der beispielsweise als Einweg-Bioreaktor mit flexibler Wandung 5 ausgebildet ist, die einen Reaktorinnenraum 6 umschließt. Der Mischerschaft 2 durchdringt dabei ein in vertikaler Richtung oben angeordneten Deckenteil 7 des Bioreaktors 4. Gegenüber dem Deckenteil 7 ist der Mischerschaft 2 abgedichtet. Der Vibrationsmischer 1 wird über einen mit dem Mischerschaft 2 außerhalb des Reaktorinnenraumes 6 verbindbaren Antrieb 8 angetrieben. Von dem Antrieb 8 wird der Mischerschaft 2 in longitudinale Bewegungen versetzt, die auf das an dem Mischerschaft 2 angeordnete Mischelement 3 übertragen werden. Das Mischelement 3 ist dabei als eine ebene Mischerplatte 9 ausgebildet, die sich konisch verjüngende Löcher 10 als eine Art Venturi-Düsen aufweist.

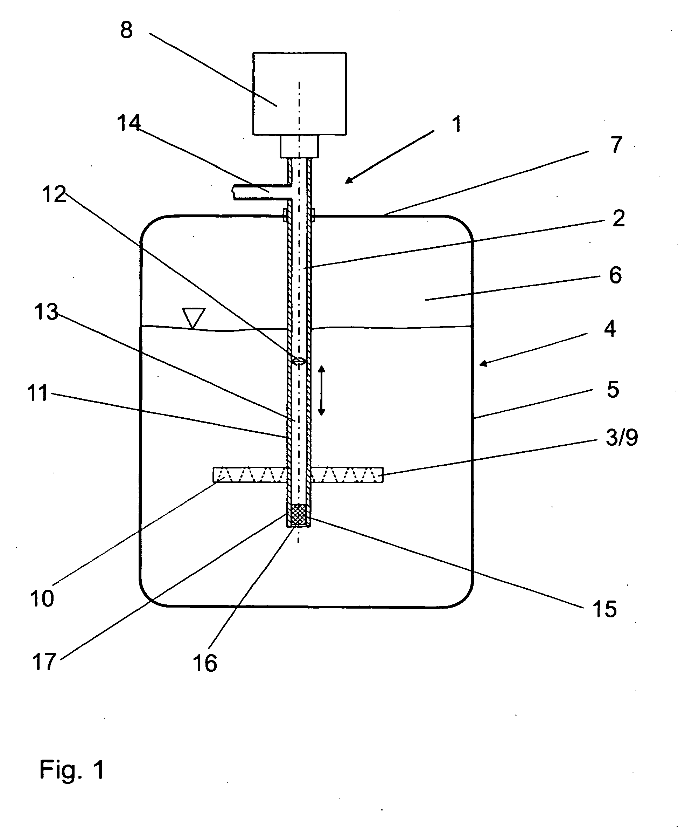

Der Mischerschaft 2 ist als ein Rohr 11 ausgebildet, dessen freies Lumen 12 einen Kanal 13 bildet. Außerhalb des Reaktorinnenraumes 6 weist der Mischerschaft 2 einen seitlichen Zugang 14 auf, der eine Verbindung zu dem Kanal 13 herstellt. An dem in vertikaler Richtung unten liegenden freien Ende des Mischerschaftes 2 ist gemäß Ausführungsbeispiel von Figur 1 kurz unterhalb des Mischelementes 3 eine Begasungseinheit 15 angeordnet, die einer zum Reaktorinnenraum 6 gerichteten Mündung 16 des Kanals 13 vorgelagert ist. Die Begasungseinheit 15 weist eine poröse Struktur auf, die geeignet ist, über den seitlichen Zugang 14 zugeführtes Gas zu filtern. Vorzugsweise besitzt die poröse Struktur eine Porengröße von weniger als 0,6 µm, bevorzugt von weniger als 0,45 µm. In einer bevorzugten Ausführungsform der Erfindung besitzt die poröse Struktur hydrophobe Eigenschaften. Dadurch wird das Eindringen von Flüssigkeiten in den Schaft 13 verhindert, wenn der Druck im Innern des Schaftes kleiner ist als im Reaktorinnenraum 6. Ohne die Begasungseinheit 15 kann über den seitlichen Zugang 14 auch ein flüssiges Medium zugeführt werden.

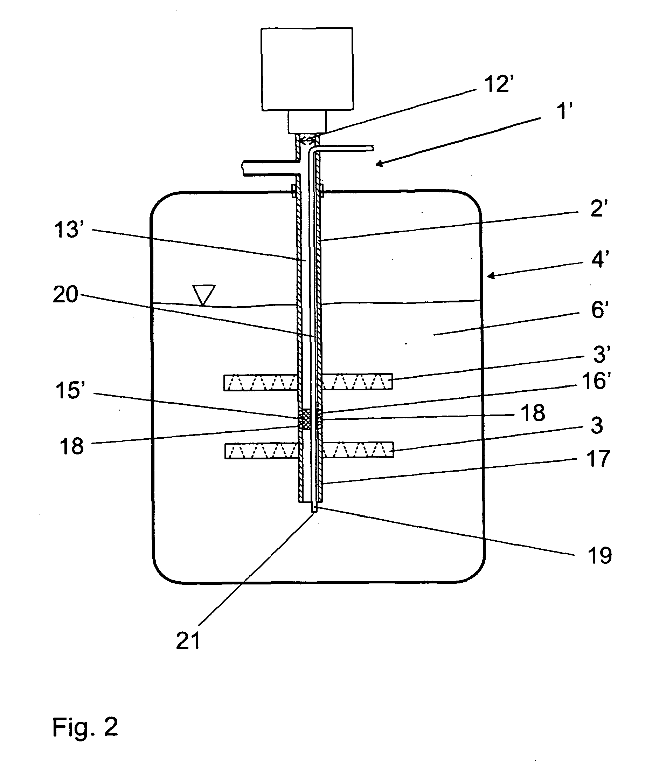

Gemäß dem Ausführungsbeispiel von Figur 2 weist der Vibrationsmischer 1' an seinem Mischerschaft 2' zwei in einem Abstand zueinander angeordnete Mischelemente 3, 3' auf. In dem Kanal 13' ist zwischen den beiden Mischelementen 3, 3' die Begasungseinheit 15' angeordnet. Im Bereich der Begasungseinheit 15' sind in der Schaftwandung 17 Öffnungen 18 angeordnet, über die von der Begasungseinheit 15' gefiltertes Gas in den Reaktorinnenraum 6' ausströmt. Damit bilden die Öffnungen 18 eine Mündung 16'. In dem freien Lumen 12' des Kanals 13' ist ein Schlauch 19 angeordnet, dessen freies Lumen einen zweiten Kanal 20 bildet. Der Schlauch 19 wird im außerhalb des Reaktorinnenraumes 6' angeordneten oberen Bereich des Mischerschaftes 2' aus diesem seitlich herausgeführt und dabei gasdicht gegenüber der Schaftwandung 17 abgedichtet. Im Bereich der Begasungseinheit 15' wird der Schlauch 19 durch diese gasdicht hindurchgeführt. Der zweite Kanal 20 mündet über seine Mündung 21 ebenfalls in den Reaktorinnenraum 6'.

Sowohl Mischerschaft 2, 2' als auch das Mischelement 3 bzw. die Mischelemente 3, 3' sind aus Kunststoff ausgebildet, die durch Schweißen oder Kleben miteinander verbunden sind. Auch der Schlauch 19 und der seitliche Zugang 14 sind aus Kunststoff ausgebildet. Der Vibrationsmischer 1, 1' kann vor oder nach Eindringen in den Reaktorinnenraum 6, 6' durch Bestrahlung sterilisiert werden.

Der Vibrationsmischer 1, 1' ist als Einwegmischer verwendbar.

## Patentansprüche

1. Vibrationsmischer (1), insbesondere Einweg-Mischer zur Verwendung in Einweg-Bioreaktoren (4, 4') mit flexibler Wandung, bestehend aus einem von einem Antrieb (8) in longitudinale Bewegungen versetzbaren Mischerschaft (2, 2') mit mindestens einem Mischelement (3, 3') und mindestens einem Kanal (13, 13') mit einer Mündung (16) zu einem Reaktorinnenraum (6, 6') hin, wobei über den mindestens einen Kanal (13, 13') Gas zuführbar ist,
**dadurch gekennzeichnet,**
**dass** der Mündung (16) eine Begasungseinheit (15, 15') vorgelagert ist oder diese die Mündung (16) bildet, wobei die Begasungseinheit (15, 15') zur Gasfilterung eine poröse Struktur aufweist.

2. Vibrationsmischer nach Anspruche 1 ,
**dadurch gekennzeichnet,**
**dass** die Mündung (16) an dem dem Antrieb abgewandten freien Ende des Mischerschaftes angeordnet ist.

3. Vibrationsmischer nach Anspruch 1,
**dadurch gekennzeichnet,**
**dass** die Begasungseinheit (15') dem ersten Mischelement (3') zum Antrieb hin vorgelagert ist.

4. Vibrationsmischer nach Anspruch 1,
**dadurch gekennzeichnet,**
**dass** die Begasungseinheit (15') zwischen zwei Mischelementen (3') am Mischerschaft (2') angeordnet ist.

5. Vibrationsmischer nach einem der Ansprüche 1 bis 4,
**dadurch gekennzeichnet,**
**dass** die Begasungseinheit (15, 15') als eine Membran ausgebildet ist.

6. Vibrationsmischer nach einem der Ansprüche 1 bis 4,
**dadurch gekennzeichnet,**
**dass** die Begasungseinheit (15, 15') als eine Kunststofffritte ausgebildet ist.

7. Vibrationsmischer nach einem der Ansprüche 1 bis 6,
**dadurch gekennzeichnet,**
**dass** in dem Mischerschaft (2') ein zweiter Kanal (20) vorgesehen ist, über den Medien zu- oder abgeführt werden können.

8. Vibrationsmischer nach einem der Ansprüche 1 bis 7,
**dadurch gekennzeichnet,**
**dass** der Mischerschaft (2, 2') als ein Rohr ausgebildet ist, dessen freies Lumen (12, 12') den ersten Kanal (13, 13') bildet.

9. Vibrationsmischer nach Anspruch 8,
**dadurch gekennzeichnet,**
**dass** in dem freien Lumen (12') des Mischerschaftes (2') ein Rohr oder ein Schlauch (19) angeordnet ist, das den zweiten Kanal (20) bildet.

## Claims

1. Vibratory mixer (1), particularly disposable mixer for use in disposable bioreactors (4, 4') with flexible walling, consisting of a mixer shaft (2, 2'), which can be set into longitudinal movements by a drive (8), with at least one mixer element (3, 3') and at least one channel (13, 13') with an opening (16) towards a reactor interior space (6, 6'), wherein gas can be fed via the at least one channel (13, 13'), **characterised in that** a gassification unit (15, 15') is mounted upstream of the opening (16) or forms this opening (16), wherein the gassification unit (15, 15') has a porous structure for gas filtration.

2. Vibratory mixer according to claim 1, **characterised in that** the opening (16) is arranged at the free end of the mixer shaft remote from the drive.

3. Vibratory mixer according to claim 1, **characterised in that** the gassification unit (15') is mounted upstream of the first mixing element (3') with respect to the drive.

4. Vibratory mixer according to claim 1, **characterised in that** the gassification unit (15') is arranged between two mixing elements (3') at the mixer shaft (2').

5. Vibratory mixer according to any one of claims 1 to 4, **characterised in that** gassification unit (15, 15') is formed as a membrane.

6. Vibratory mixer according to any one of claims 1 to 4, **characterised in that** the gassification unit (15, 15') is formed as a plastics material frit.

7. Vibratory mixer according to any one of claims 1 to 6, **characterised in that** a second channel (20) by way of which media can be fed or discharged is provided in the mixer shaft (2').

8. Vibratory mixer according to any one of claims 1 to 7, **characterised in that** the mixer shaft (2, 2') is constructed as a tube, the free lumen (12, 12') forms the first channel (13, 13').

9. Vibratory mixer according to claim 8, **characterised in that** a tube or a hose (19) forming the second channel (20) is arranged in the free lumen (12') of the mixer shaft (2').

## Revendications

1. Mélangeur par vibrations (1), en particulier mélangeur à usage unique destiné à être utilisé dans des bioréacteurs (4, 4') à usage unique avec paroi flexible, formé par une tige (2, 2'), apte à être animée de mouvements longitudinaux au moyen d'un système d'entraînement (8) et comportant au moins un élément mélangeur (3, 3') et au moins un conduit (13, 13') avec une bouche (16) menant vers l'enceinte intérieure (6, 6') du réacteur, un gaz pouvant être acheminé via ledit au moins un conduit (13, 13'), **caractérisé en ce qu'**une unité d'introduction de gaz (15, 15') est montée en amont de la bouche (16) ou est formée par la bouche (16), l'unité d'introduction de gaz (15, 15') comportant une structure poreuse en vue du filtrage du gaz.

2. Mélangeur par vibrations selon la revendication 1, **caractérisé en ce que** la bouche (16) est agencée au niveau de l'extrémité libre de la tige, opposée au système d'entraînement.

3. Mélangeur par vibrations selon la revendication 1, **caractérisé en ce que** l'unité d'introduction de gaz (15') est montée en amont, par rapport au système d'entraînement, du premier élément mélangeur (3').

4. Mélangeur par vibrations selon la revendication 1, **caractérisé en ce que** l'unité d'introduction de gaz (15') est disposée sur la tige (2') du mélangeur entre deux éléments mélangeurs (3').

5. Mélangeur par vibrations selon l'une quelconque des revendications 1 à 4, **caractérisé en ce que** l'unité d'introduction de gaz (15, 15') est réalisée sous la forme d'une membrane.

6. Mélangeur par vibrations selon l'une quelconque des revendications 1 à 4, **caractérisé en ce que** l'unité d'introduction de gaz (15, 15') est réalisée sous la forme d'une fritte en matière plastique.

7. Mélangeur par vibrations selon l'une quelconque des revendications 1 à 6, **caractérisé en ce que** dans la tige (2') du mélangeur est prévu un deuxième conduit (20), par l'intermédiaire duquel les fluides peuvent être admis et évacués.

8. Mélangeur par vibrations selon l'une quelconque des revendications 1 à 7, **caractérisé en ce que** la tige (2, 2') du mélangeur est réalisée sous la forme d'un tube, dont la lumière (12, 12') libre forme le premier conduit (13, 13').

9. Mélangeur par vibrations selon la revendication 8, **caractérisé en ce que** dans la lumière (12') libre de la tige (2') du mélangeur est disposé un tube ou un tuyau flexible (19) qui forme le deuxième conduit (20).
